# EUROPEAN PATENT SPECIFICATION

(11) **EP 2 022 443 B1**
(45) Date of publication and mention of the grant of the patent: **22.09.2010**
(21) Application number: 08160957.0
(22) Date of filing: 23.07.2008
(51) Int. Cl.: A61C 8/00

(54) **Endosseous dental implant**
Enossales Zahnimplantat
Implant dentaire endo-osseux

(30) Priority: 02.08.2007 IT BO20070555
(43) Date of publication of application: 11.02.2009
(73) Proprietor: Pidala', Daniele, 40129 Bologna (IT); Prandini, Federico, 40139 Bologna (IT)
(72) Inventor: Pidala', Daniele, 40129 Bologna (IT); Prandini, Federico, 40139 Bologna (IT)
(74) Representative: Lanzoni, Luciano

(56) References cited:
- EP-B1- 0 282 789
- US-A1- 2006 180 647
- US-A1- 2006 271 199

## Description

This invention refers to an endosseous dental implant.

The known types of endosseous dental implants comprise a hollow screw which is inserted in a respective hole cut in the upper maxilla or the lower mandible of a human being.

In detail, the hollow screw is substantially inserted partially or completely in the bone.

Once the hole has been made, it can in most cases be threaded to ensure stable coupling with the screw which, again in most cases, is threaded externally so that it can be firmly screwed into the hole.

The hollow screw also presents an inner space which is usually, but not necessarily, provided with coupling means which can also consist of partial threading.

Once inserted in the bone, the screw (technically defined in the sector jargon as an "implant") is coupled with an abutment designed to support a prosthetic appliance such as, for example, a single dental crown or a plurality of crowns connected together to generically define a so-called "prosthesis".

The abutment comprises a body which can, in some solutions, be at least partially threaded, while in other solutions quick-coupling elements may be present, and it can be inserted in the inner space of the hollow screw to connect the screw to the prosthesis.

In detail, the body of the abutment is attached to the inner space of the hollow screw by screwing or, in some very particular cases, by interference.

The known types of implants also comprise anti-rotation means positioned between the hollow screw and the abutment to prevent rotation between these two components.

These anti-rotation means can comprise a blocking element positioned on the abutment and having a polygonal-shaped cross-section, for example hexagonal or octagonal, or another geometrical shape, for example conical for certain types of particular applications.

When the abutment is screwed into the hollow screw, the blocking element is housed in a counter-shaped housing close to the head of the hollow screw.

In this way, the abutment coupled with the hollow screw cannot rotate with respect to the screw.

There is naturally a close correlation between the hollow screw and the abutment: in other words, there must be a precise correspondence at least between the inner diameter of the hollow screw and the diameter of the coupling body, for example, if present, the type and pitch of the threading, the geometrical shape of the blocking element, etc.

The abutment also comprises a support portion for attachment to the dental crown or plurality of crowns. The support portion is rigidly fixed to the body attached to the hollow screw.

In use, the hole in the bone is made by using appropriate drills or burs. In the most usual case of threading, the hole is then threaded with a tap corresponding to the hollow screw to be implanted.

Once the screw has been inserted in the hole, it is necessary to wait for a period of time to allow the bone tissue to integrate with the screw so that the screw is firmly fixed within the bone (during this period of time, the hollow screw is temporarily closed by a so-called "healing screw").

This consolidation step is prolonged and can last for several weeks or months.

It is known from document EP 0 282 789 a dental implant having a screw and a prosthetic element which is coupled with said screw. Moreover, it is known from document US 2006/180647 the use of an RFID chip in a tooth as a dental implant.

A drawback of known types of endosseous dental implants is that when a screw is inserted in the bone, it is no longer recognizable since it becomes part of the patient's bone and tissue.

In particular, it is no longer possible to identify the internal diameter of the hollow screw, the type and pitch of the threading and the shape of the housing of the blocking element.

This information is indispensable in order to identify the correct abutment to be used even after a considerable period of time.

In order to preserve this information, the patient is given a document indicating at least the name of the manufacturer and the model of the implanted screw.

This document does not, however, guarantee that the information will be maintained as it may be thrown away or lost.

It should be noted that if this information is not available when the abutment to be attached to the screw has to be chosen, then it is impossible to make this choice and the previously inserted screw must be removed.

This operation is fairly complicated and expensive since it involves drilling the portion of bone comprising the screw and eliminating the removed screw.

In this scenario, the aim of this invention is to propose an endosseous dental implant that does not have the above-mentioned drawbacks.

In particular, the aim of this invention is to provide an endosseous dental implant which can be recognized even a considerable period of time after its insertion.

A further aim of this invention is propose an endosseous dental implant that can be recognized easily and quickly.

According to the invention, the technical purpose and the described aims are achieved by an endosseous dental implant with the technical characteristics described in one or more of the accompanying claims. The technical characteristics of the invention, according to the above-mentioned aims, can be clearly identified in the claims listed below, and the advantages of the invention will become more evident in the detailed description which follows and which refers to the accompanying drawings which represent one non-binding example of an embodiment, in which:

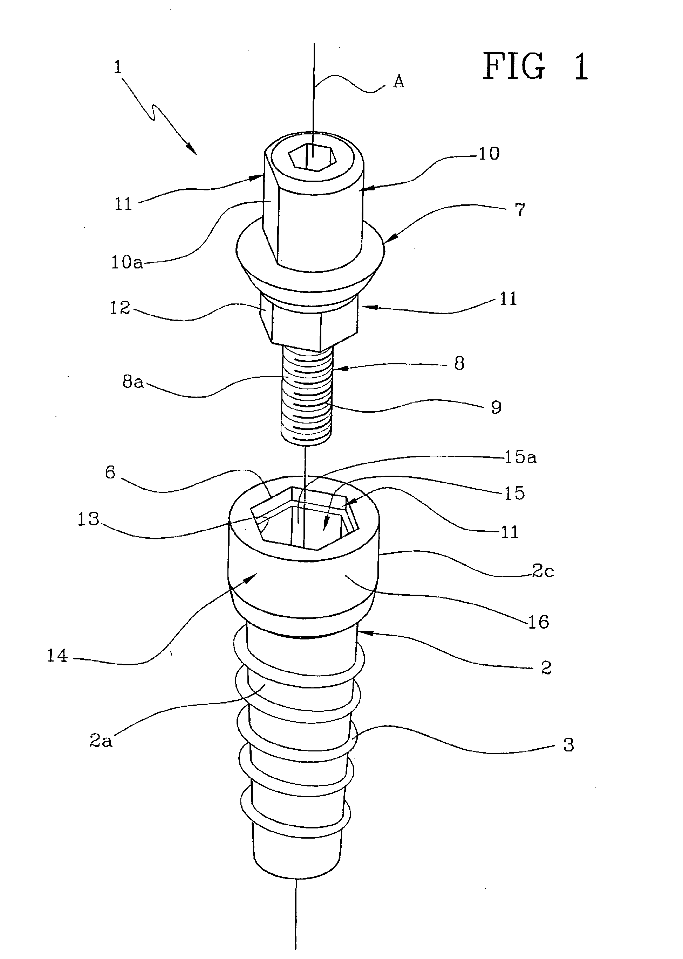

- figure 1 shows a prospective exploded view of an endosseous dental implant according to this invention;
- figure 2 shows a cross-section of a detail of the dental implant in figure 1;
- figures 3 and 4 show respective alternative embodiments of the detail in figure 2, wherein figure 4 does not form part of the claimed invention; and
- figure 5 shows a schematic view of a detail of the dental implant according to this invention.

In accordance with the accompanying drawings, the reference number 1 indicates overall an endosseous dental implant according to this invention.

The dental implant 1 comprises a hollow screw 2 (known also by the term of fixture) which can be inserted in a hole made in a human bone.

In particular, the hole can be made in the upper maxilla (or more simply, maxilla) or in the lower mandible (or more commonly, mandible) of a human being.

This solution is the most common one in the endodontic implant field and foresees coupling systems with threadings which do not, however, in any way limit the solution in question if there are other types of coupling means.

The hollow screw 2 presents an outer surface 2a equipped with first coupling means 3 (for example, respective threading 3) to couple the screw 2 with the bone, and an inner surface 2b which defines an inner space 4. The inner surface 2b is also equipped with second coupling means 5 (for example, respective threading 5) to allow the hollow screw 2 to be coupled with a prosthesis (not shown in the figures).

By way of example, the prosthesis is a single dental crown made from ceramic or synthetic material or a plurality of dental crowns rigidly connected to each other.

The inner space 4 is also defined by a conically shaped base surface 4a. Alternatively, the base surface 4a can be flat, depending on the type of tool used when creating the inner space 4. An opening 6 located at one end 2c of the screw 2 and facing the outside of the bone provides access to the inner space 4.

In detail, the hole made in the bone is threaded by means of a tap and the screw 2 is screwed into the hole so that the said end 2c of the screw 2 is aligned with the surface of the bone.

The dental implant 1 also comprises an abutment 7 which can be coupled with the screw 2.

The abutment 7 comprises a connecting body 8, which can be inserted in the inner space 4 of the screw 2 and can be cylindrical and comprises third coupling means 9 (respective threading 9) on its side surface 8a to allow it to engage with the threading 5 on the inner surface 2b of the screw.

The abutment 7 also comprises a support portion 10 on which the prosthesis is fixed and which is rigidly attached to the connecting body 8.

The dental implant 1 can also comprise anti-rotation means 11 located on the screw 2 and on the abutment 7 to prevent rotation between at least these two components.

The anti-rotation means 11 can comprise a blocking element 12 on the abutment 7 between the connecting body 8 and the support portion 10.

The blocking element 12 presents a cross-section at a longitudinal axis "A" of the abutment 7 with at least one straight side. The cross-section is preferably polygonal in shape. By way of example, in the described embodiment this cross-section is hexagonal. In other words, the blocking element 12 presents the shape of a prism with a hexagonal base. Alternatively, the cross-section can be octagonal or another geometrical shape.

The blocking element 12 is accommodated in a counter-shaped housing 13 cut in the head 14 of the screw 2. This housing 13 is close to the opening 6 providing access to the inner space 4.

More specifically, the head 14 of the screw 2 presents an inner surface 15, defining the said housing 13, with at least one flat portion 15a. In the described embodiment, this inner surface 15 presents a hexagonal cross-section.

The head 14 also presents a substantially cylindrical outer surface 16 to facilitate insertion of the screw 2 into the hole in the bone.

When the abutment 7 is coupled with the screw 2, the abutment 7 is screwed into the inner space 4 until the blocking element 12 is accommodated in the housing 13 in the head 14.

In the embodiment shown in the drawings, merely by way of example, since the housing 13 is counter-shaped to the blocking element 12, the abutment 7 cannot rotate with respect to the screw 2.

The anti-rotation means 11 can also consist of a flat portion 10a cut in the support portion 10 of the abutment 7 to prevent the prosthesis rotating with respect to the abutment 7.

The inner space 4 of the screw 2 and the connecting body 8 of the abutment 7 must obviously correspond perfectly.

In particular, the geometry of the threadings 5, 9, cut respectively on the inner surface 2b of the screw 2 and on the connecting body 8, the diameter and length of the inner space 4 in the screw 2 and of the connecting body 8, the shape of the blocking element 12 and of the housing 13 of the anti-rotation means 11 must all correspond perfectly.

The dental implant 1 also comprises and electronic identification device 17 which is fitted on the screw 2.

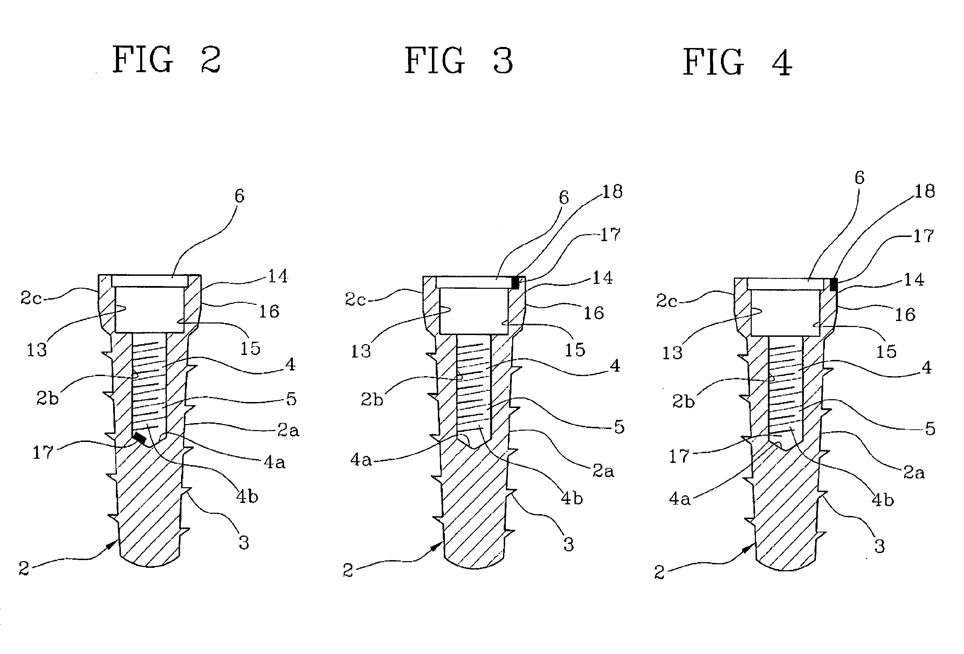

With reference to figure 2, the electronic identification device 17 is positioned in the inner space 4 of the screw 2. More specifically, the electronic identification device 17 is attached to the base surface 4a of the inner space 4, for example by glueing.

Advantageously, in this embodiment, the electronic identification device 17 occupies a blind area 4b of the inner space 4 defined by an undercut area of a tool used to form the inner space 4.

Since the blind area 4b remains unused even after the coupling of the screw 2 and the abutment 7, positioning the electronic identification device 17 on the base surface 4b of the inner space 4 means that it is not necessary to create additional housings to accommodate the device 17.

In the embodiment shown in figure 3, the electronic identification device 17 is positioned in a notch 18 cut in the inner surface 15 of the head 14 of the screw 2. In particular, the notch 18 is cut close to a flat portion 15a of the inner surface 15. In the example shown in figure 4, which does not form part of the invention, the electronic identification device 17 is positioned in a notch 18 cut in the outer surface 16 of the head 14 of the screw 2.

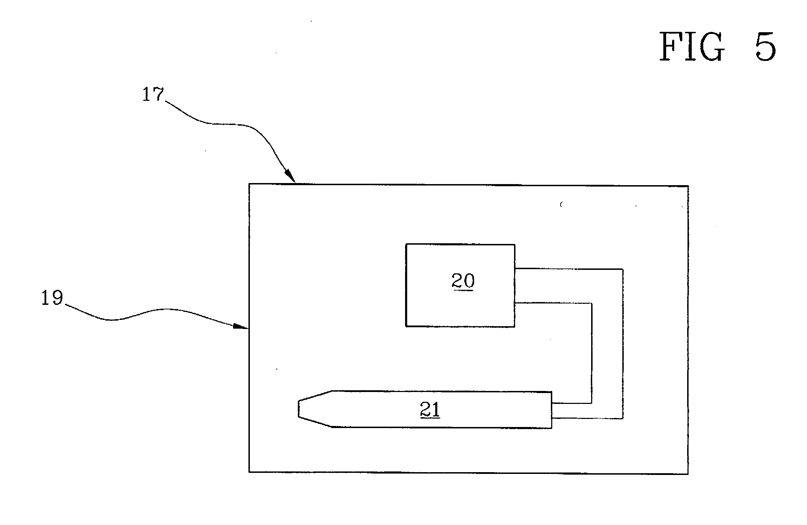

The electronic identification device 17 can preferably be the RFID (*Radio Frequency Identification*) type and comprises a transponder 19 consisting of a permanent memory 20 to preserve at least one piece of information and a unit 21 connected to the memory 20 to transmit this information and which can consist of an antenna (see figure 5).

In a more simplified alternative, the transmission unit 21 can be the implant screw 2 itself.

This information represents at least one identification parameter of the dental implant 1, such as for example the make and/or model of the dental implant 1.

To make the task of identification easier, this information can be in the form of a serial alphanumeric code that, by means of a relative external data base, contains all the information relative to the dental implant 1.

The permanent memory 20 can, however, contain other information relative, for example, to the date on which the dental implant 1 was installed, the name of the operator who fitted the implant and/or additional clinical notes: this makes it possible to immediately obtain a so-called "implant record" with all the relative details.

In particular, the permanent memory 20 is the read-write type.

The transponder 19 is the passive type. In other words, the transponder 19 does not comprise its own sources of electrical power supply.

Within the sphere of the RFID technology, there are some standards in relation to the frequencies used to transmit information. As far as passive transponders are concerned, these usually transmit at 125 kHz or 13.56 MHz.

In the embodiments described herein, the transponder 19 can use the frequency of 13.56 MHz.

In use, when it is necessary to identify the type of dental implant 1 fitted in a patient, the operator uses a special reader (not shown) which sends an electromagnetic signal to interrogate the electronic identification device 17.

The electromagnetic signal activates the transponder 19 which responds by sending another electromagnetic signal containing the information stored in the permanent memory 20.

The invention fulfils the proposed aims and provides significant advantages.

The dental implant 1 according to this invention is recognizable even a considerable period of time after being implanted in the patient.

In fact, especially as regards the screw 2, although this continues to remain completely out of sight (and thus not recognizable to the naked eye), its recognition is immediate since this occurs by means of the electronic identification device 17 with the above-mentioned reader.

It is therefore possible to always identify at least the make and model of the dental implant 1 installed and, thus, all the parameters of the screw 2 necessary to identify the appropriate abutment 7 to be coupled with the screw 2.

This also eliminates the need to preserve paper documents containing this information, which can easily be damaged or lost.

In addition, the recognition of this information is extremely simple and fast since a single interrogation operation immediately provides all the information contained in the electronic identification device 17.

The dental implant 1 according to this invention is also extremely flexible as regards the type of additional information that can be memorised and stored in the electronic identification device 17, as mentioned above.

The invention as described above is susceptible to evident industrial application; it can also be the subject of numerous modifications and variations, all within the scope of its disclosure; all the components can also be replaced with technically equivalent elements.

## Claims

1. A dental implant comprising a hollow screw (2) which can be inserted in a hole made in a human bone; said hollow screw (2) presenting an outer surface (2a) provided with first means (3) for coupling said screw (2) to said bone, and an inner surface (2b) provided at least in part with second means (5) for coupling said screw (2) to a prosthetic appliance; said inner surface (2b) defining at least in part an inner space (4), **characterised in that** it comprises an electronic identification device (17) fixed to said hollow screw (2) and located in said inner space (4) of said hollow screw (2).

2. A dental implant according to claim 1, **characterised in that** said electronic identification device (17) is positioned on a base surface (4a) of said inner space (4) of said hollow screw (2).

3. A dental implant according to claim 1, **characterised in that** said hollow screw (2) comprises a head (14) positioned close to an opening (6) of said inner space (4); said head (14) presenting an inner surface (15) with at least one flat portion (15a) to prevent relative rotations of said prosthetic appliance.

4. A dental implant according to claim 3, **characterised in that** said electronic identification device (17) is positioned in a notch (18) cut in said inner surface (15) of said head (14).

5. A dental implant according to claim 4, **characterised in that** said notch (18) is cut in a flat portion (15a) of said inner surface (15) of said head (14).

6. A dental implant according to any of the foregoing claims, **characterised in that** said electronic identification device (17) comprises a transponder (19); said transponder (19) comprising a permanent memory (20) to preserve at least one piece of information and a unit (21) designed to transmit this information.

7. A dental implant according to claim 6, **characterised in that** said unit (21) designed to transmit this information is said hollow screw (2).

8. A dental implant according to claim 6, **characterised in that** said information is represented by at least one identification parameter of said dental implant.

9. A dental implant according to claim 8, **characterised in that** said identification parameter is relative to the make and/or model of said dental implant.

10. The use of an electronic identification device (17) connected to an inner space (4) of a hollow screw (2) of a dental implant (1) fixed in a human bone to acquire at least one piece of information relative to the make and/or model of said dental implant (1).

## Patentansprüche

1. Zahnimplantat, beinhaltend eine Hohlschraube (2) zum Einsetzen in eine in einem menschlichen Knochen angefertigte Öffnung; wobei die Hohlschraube (2) eine äußere Oberfläche (2a) aufweist, die mit ersten Mitteln (3) zur Verbindung der Schraube (2) mit dem Knochen versehen ist, und eine innere Oberfläche (2b) aufweist, die zumindest teilweise mit zweiten Mitteln (5) zur Verbindung der Schraube (2) mit einem prothetischen Element versehen ist; wobei die innere Oberfläche (2b) zumindest teilweise einen Innenraum (4) bildet; **dadurch gekennzeichnet, dass** es eine elektronische Identifikationsvorrichtung (17) beinhaltet, die an der Hohlschraube (2) befestigt und in dem Innenraum (4) der Hohlschraube (2) angeordnet ist.

2. Zahnimplantat nach Anspruch 1, **dadurch gekennzeichnet, dass** die elektronische Identifikationsvorrichtung (17) auf einer Basisoberfläche (4a) des Innenraumes (4) der Hohlschraube (2) angeordnet ist.

3. Zahnimplantat nach Anspruch 1, **dadurch gekennzeichnet, dass** die Hohlschraube (2) einen Kopf (14) beinhaltet, der nahe einer Öffnung (6) des Innenraumes (4) angeordnet ist; wobei der Kopf (14) eine innere Oberfläche (15) mit zumindest einem flachen Abschnitt (15a) zur Verhinderung relativer Rotationsbewegungen des prothetischen Elementes aufweist.

4. Zahnimplantat nach Anspruch 3, **dadurch gekennzeichnet, dass** die elektronische Identifikationsvorrichtung (17) in einer Einkerbung (18) angeordnet ist, die in der inneren Oberfläche (15) des Kopfes (14) eingeschnitten ist.

5. Zahnimplantat nach Anspruch 4, **dadurch gekennzeichnet, dass** die Einkerbung (18) in einem flachen Abschnitt (15a) der inneren Oberfläche (15) des Kopfes (14) eingeschnitten ist.

6. Zahnimplantat nach einem der vorhergehenden Ansprüche, **dadurch gekennzeichnet, dass** die elektronische Identifikationsvorrichtung (17) einen Transponder (19) beinhaltet; wobei der Transponder (19) einen permanenten Speicher (20) zum Aufbewahren zumindest einer Information und eine Einheit (21) für die Übertragung dieser Information beinhaltet.

7. Zahnimplantat nach Anspruch 6, **dadurch gekennzeichnet, dass** die Einheit (21) zur Übertragung der Information die genannte Hohlschraube (2) ist.

8. Zahnimplantat nach Anspruch 6, **dadurch gekennzeichnet, dass** die Information durch zumindest einen Identifikationsparameter des Implantats dargestellt ist.

9. Zahnimplantat nach Anspruch 8 **dadurch gekennzeichnet, dass** der Identifikationsparameter auf die Marke und/oder das Modell des Zahnimplantats bezogen ist.

10. Gebrauch einer elektronischen Identifikationsvorrichtung (17), die mit einem Innenraum (4) einer Hohlschraube (2) eines Zahnimplantats (1) verbunden ist, das in einem menschlichen Knochen fixiert ist, um zumindest eine Information zu erfassen, die auf die Marke und/oder das Modell des Zahnimplantats (1) bezogen ist.

## Revendications

1. Un implant dentaire comprenant une vis creuse (2) qui peut être introduite dans un trou pratiqué dans un os humain ; ladite vis creuse (2) présentant une surface extérieure (2a) dotée de premiers moyens (3) pour l'accouplement de ladite vis (2) avec ledit os, et une surface intérieure (2b) dotée au moins en partie de deuxièmes moyens (5) pour l'accouplement de ladite vis (2) avec un appareil de prothèse ; ladite surface intérieure (2b) définissant au moins en partie un espace intérieur (4), ledit implant dentaire étant **caractérisé en ce qu'**il comprend un dispositif électronique d'identification (17) fixé à ladite vis creuse (2) et situé dans ledit espace intérieur (4) de ladite vis creuse (2).

2. Un implant dentaire selon la revendication 1, **caractérisé en ce que** le dispositif électronique d'identification (17) est positionné sur une surface de base (4a) dudit espace intérieur (4) de ladite vis creuse (2).

3. Un implant dentaire selon la revendication 1, **caractérisé en ce que** ladite vis creuse (2) comprend une tête (14) positionnée à proximité d'une ouverture (6) dudit espace intérieur (4) ; ladite tête (14) présentant une surface intérieure (15) ayant au moins une partie plate (15a) afin d'empêcher des rotations relatives dudit appareil de prothèse.

4. Un implant dentaire selon la revendication 3, **caractérisé en ce que** ledit dispositif électronique d'identification (17) est positionné dans une encoche (18) découpée dans ladite surface intérieure (15) de ladite tête (14).

5. Un implant dentaire selon la revendication 4, **caractérisé en ce que** ladite encoche (18) est découpée dans une partie plate (15a) de ladite surface intérieure (15) de ladite tête (14).

6. Un implant dentaire selon l'une quelconque des revendications précédentes, **caractérisé en ce que** ledit dispositif électronique d'identification (17) comprend un transpondeur (19) ; ledit transpondeur (19) comprenant une mémoire permanente (20) pour conserver au moins une information et une unité (21) destinée à transmettre cette information.

7. Un implant dentaire selon la revendication 6, **caractérisé en ce que** ladite unité (21) destinée à transmettre cette information est définie par ladite vis creuse (2).

8. Un implant dentaire selon la revendication 6, **caractérisé en ce que** ladite information est représentée par au moins un paramètre d'identification dudit implant dentaire.

9. Un implant dentaire selon la revendication 8, **caractérisé en ce que** ledit paramètre d'identification est relatif à la marque et/ou au modèle dudit implant dentaire.

10. Utilisation d'un dispositif électronique d'identification (17) relié à un espace intérieur (4) d'une vis creuse (2) d'un implant dentaire (1) fixé dans un os humain pour acquérir au moins une information relative à la marque et/ou au modèle dudit implant dentaire (1).
